# EUROPÄISCHE PATENTANMELDUNG

(11) **EP 0 979 874 A2**
(43) Veröffentlichungstag der Anmeldung: **16.02.2000**
(21) Anmeldenummer: 99115160.6
(22) Anmeldetag: 13.08.1999
(51) Int. Cl.: C12N 15/54, C12N 15/82, C12N 9/10, C12N 5/10, A01H 5/00, A01H 5/10

(54) **Protein und DNA-Sequenz der Pinosylvin-3-0-Methyltransferase (PMT)**

(30) Priorität: 13.08.1998 DE 19836774
(71) Anmelder: GSF-Forschungszentrum für Umwelt und Gesundheit, GmbH, 85764 Oberschleissheim (DE)
(72) Erfinder: Heller, Werner, Dr., 85386 Eching (DE); Sandermann, Heinrich, Prof. Dr., 81377 München (DE); Ernst, Dietrich, Dr., 82131 Gauting (DE); Drouet, Alain, Dr., F-45160 Olivet (FR); Chiron, Helene, 85716 Unterschleissheim (DE)
(74) Vertreter: Reinhard - Skuhra - Weise & Partner

(57) **Zusammenfassung**

Die vorliegende Erfindung betrifft Pinosylvin-3-O-methyltransferase-Gene, neue DNA-Sequenzen, die für die Pinosylvin-3-O-methyltransferase aus Pinus sylvestris kodieren sowie hiervon abgeleitete Aminosäuresequenzen und die Verwendung dieser DNA-Sequenzen zur Erzeugung transgener Pflanzen, die gegen Pathogene resistent sind.

## Beschreibung

Die vorliegende Erfindung betrifft Pinosylvin-3-O-methyltransferase-Gene, die DNA-Sequenz sowie die davon abgeleitete Aminosäuresequenz der Pinosylvin-3-O-methyltransferase (PMT) sowie hiervon abgeleitete Neuentwicklungen.

Pinus sylvestris akkumuliert bei Einwirkung verschiedener Streßfaktoren sowohl Pinosylvin (PS) als auch den Monomethylether von Pinosylvin (PSM). Es ist bekannt, daß PSM Pflanzen vor verschiedenen Streßfaktoren wesentlich wirksamer schützt als PS. Beispiele hierfür sind der Schutz vor pathogenen Organismen wie beispielsweise Pilzen (Lieutier et al., 1996) und gegen Ozon (Rosemann et al., 1991). Weiterhin ist bekannt, daß PMT durch Ozon wesentlich stärker induzierbar ist als die Stilbensynthase (STS) (Trost, 1994). Deshalb ist auch die Regulation der Aktivität der Pinosylvin-3-O-methyltransferase von eminent biologischer und auch wirtschaftlicher Bedeutung.

Methyltransferasen sind an sich bekannt. Sie katalysieren die Übertragung von Methylgruppen von einer Verbindung auf eine andere. Bekannt sind weiterhin pflanzliche O-Methyltransferasen, die eine Methylierung am Sauerstoffatom bewirken. Beispiele hierfür sind die O-Methyltransferase aus Pinus radiata (U70873), die Kaffeinsäure-O-methyltransferase aus Pinus taeda (U39301), die Catechol-O-methyltransferase auf Nicotiana tabacum (X74452) sowie die Lignin-bispezifische Säure/5-Hydroxyferulasäure-O-methyltransferase aus Populus tremoides (X62096).

In der Dissertation von Trost, LMU-München, 1994, wurde die mögliche Existenz der Aktivität einer Pinosylvin-3-O-methyltransferase (PMT) aus Kiefer (Pinus sylvestris) beschrieben. Das Enzym selbst lag jedoch nicht in gereinigter, isolierter Form vor, sondern lediglich in einem Gemisch, verunreinigt mit anderen Proteinen. Bekannt waren lediglich 3 Peptidsequenzen, die über eine zweidimensionale elektrophoretische Auftrennung erhalten wurden und die viele weitere verunreinigende Proteine zeigten. Nicht beschrieben und nicht isoliert waren somit sowohl das Enzyn selbst als auch das Gen, das für eine Pinosylvin-3-O-methyltransferase kodiert. Unbekannt waren die cDNA und die Aminosäuresequenz der Pinosylvin-3-O-methyl-transferase, insbesondere die aus Pinus sylvestris. Die Analyse der cDNA-Sequenz und der Aminosäuresequenz von PMT, z.B. aus Pinus sylvestris, ist eine unabdingbare Voraussetzung, um molekularbiologische Strategien zur Erzeugung von transgenen Pflanzen zu entwickeln, die z.B. gegen pathogene Organismen widerstandsfähiger sind. Es ist somit eine wichtige Aufgabe der vorliegenden Erfindung, Pinosylvin-3-O-methyltransferase-Gene bereitzustellen.

Diese Aufgabe wird erfindungsgemäß durch die Bereitstellung von Pinosylvin-3-O-methyltransferase-Genen, bevorzugt aus Pinus sylvestris, gelöst.

PMT-Nukleinsäure- und Aminosäuresequenzen sind bisher nicht bekannt. Bekannt sind lediglich OMT-Sequenzen. Ein Homologievergleich mit bekannten OMT-Sequenzen aus Datenbanken wurde mit dem Blast-Programm durchgeführt. Eine vergleichende Vollängenanalyse mit ausgewählten OMT-Sequenzen ergab eine maximale Identität von ca. 45% zu beispielsweise Flavonoid-OMTs.

Es ist eine weitere wichtige Aufgabe der vorliegenden Erfindung, neue DNA-Sequenzen bereitzustellen, die für eine Pinosylvin-3-O-methyltransferase, z.B. aus Pinus sylvestris, kodieren.

Eine weitere Aufgabe der Erfindung besteht darin, die von dieser DNA-Sequenz abgeleitete Aminosäuresequenz aufzuzeigen.

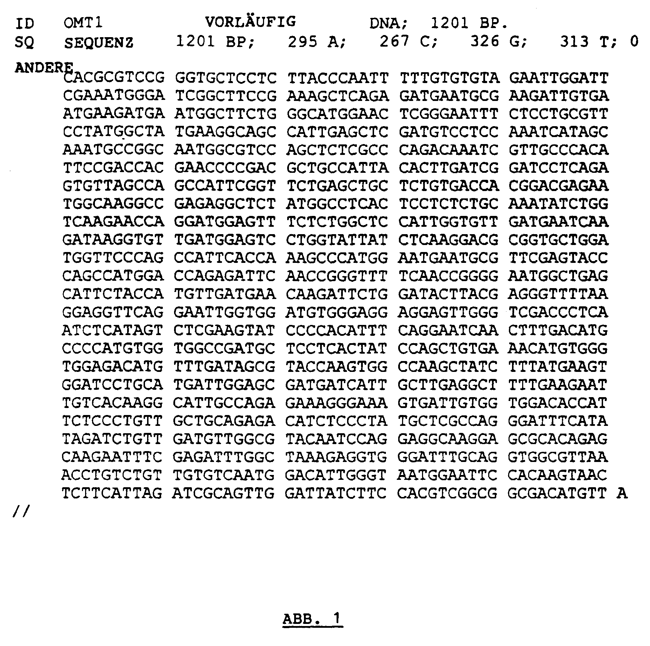

Diese Aufgabe wird erfindungsgemäß durch eine gereinigte und isolierte DNA mit der in Figur 1 dargestellten PMT-Sequenz, die für ein pflanzliches PMT-Protein, bevorzugt aus Pinus sylvestris, kodiert, gelöst.

Von der Erfindung mitumfaßt werden auch Teile dieser gereinigten DNA, die für ein biologisch aktives PMT-Protein kodieren sowie funktionelle Teile der DNA, die für das hierfür kodierte Protein charakteristisch sind. Unter "charakteristisch" sind solche Teile zu verstehen, die ausschließlich in PMT-Proteinen vorkommen; unter "funktionell" sind Teile der PMT-Sequenz mit spezifischen Funktionen zu verstehen, beispielsweise Promotor, Enhancer und andere Steuersequenzen.

Die vorliegende Erfindung umfaßt auch DNA, die mit der in Figur 1 dargestellten PMT-Sequenz unter stringenten, bevorzugt hochstringenten, Standard-Bedingungen hybridisierbar ist sowie funktionelle Teile dieser DNA, die beispielsweise für ein pflanzliches, biologisch aktives PMT-Protein kodieren.

Der Begriff "Hybridisierung" bedeutet im Rahmen dieser Erfindung eine Hybridisierung unter konventionellen Hybridisierungsbedingungen, vorzugsweise unter hochstringenten Bedingungen, wie sie beispielsweise in Sambrook et al. (1989) Molecular Cloning: a Laboratory Manual, Cold Spring Harbor Laboratory Press, New York, beschrieben sind. Hierzu werden beispielsweise eine Hybridisierungstemperatur von 65°C, ein Puffer mit 6 x SSC, 5x Denhard's, 0,5% SDS und 100 µg/ml Herings-DNA und Waschbedingungen von 0,5 x SSC/0,5% SDS bei 65°C gewählt (für ³²P-markierte Southern-Hybridisierungen.

Erfindungsgemäß werden auch solche DNA-Sequenzen beansprucht, die aufgrund der Degeneration des genetischen Codes für die selbe Aminosäuresequenz kodieren wie die DNA-Sequenzen, die oben näher beschrieben wurden.

Bei der erfindungsgemäß bereitgestellten DNA handelt es sich um eine cDNA. Umfaßt von der vorliegenden Erfindung wird auch genomische DNA mit den Intron-Sequenzen. Ausgehend von der erfindungsgemäß bereitgestellten cDNA kann ein Fachmann ohne erfinderisches Bemühen die genomische DNA analysieren

Von der Erfindung umfaßt werden auch RNA-Moleküle, beispielsweise mRNA-Moleküle, die sich von der oben näher beschriebenen und in den Ansprüchen gekennzeichneten DNA ableiten.

Die mit der erfindungsgemäßen DNA hybridisierenden DNA-Moleküle umfassen auch Fragmente, Derivate und allelische Varianten der oben beschriebenen DNA-Moleküle mit der Fähigkeit, für einen funktionellen Teil des PMT-Gens, z.B. für ein biologisch aktives PMT-Protein, zu kodieren. Der Ausdruck "Derivat" bedeutet in diesem Zusammenhang, daß die Sequenzen dieser Moleküle sich von den Sequenzen der oben beschriebenen Nukleinsäuremoleküle an einer oder mehreren Positionen unterscheiden und einen Grad an Homologie zu diesen Sequenzen aufweisen, der bei über 80, bevorzugt über 90% und besonders bevorzugt über 95% liegt. Die Abweichungen können dabei durch Deletion, Addition, Substitution, Insertion oder Rekombination entstanden sein.

Bei den Nukleinsäuremolekülen, die homolog zu den oben beschriebenen Molekülen sind und Derivate dieser Moleküle darstellen, handelt es sich in der Regel um Variationen dieser Moleküle, die Modifikationen darstellen, die diesselbe biologische Funktion ausüben. Es kann sich dabei sowohl um natürlicherweise auftretende Variationen handeln oder um Mutationen, wobei diese Modifikationen auf natürliche Weise aufgetreten sein können oder durch gezielte Mutagenese eingeführt wurden. Ferner kann es sich hierbei um synthetisch hergestellte Sequenzen handeln. Bei den allelischen Varianten kann es sich sowohl um natürlich auftretende als auch um synthetisch hergestellte oder durch rekombinante DNA-Techniken erzeugte Varianten handeln.

Erfindungsgemäß werden auch rekombinante DNA-Klonierungsvehikel bereitgestellt, die die oben näher definierte DNA enthalten. Hierbei kann es sich beispielsweise um einen Vektor handeln, der das von der DNA kodierte Protein exprimiert.

Die erfindungsgemäße, rekombinante DNA kann aber auch direkt in das Chromosom einer Wirtszelle integriert werden. Alternativ liegt es extrachromosomal auf einem Vehikel, beispielsweise auf einem Plasmid, Cosmid etc. vor.

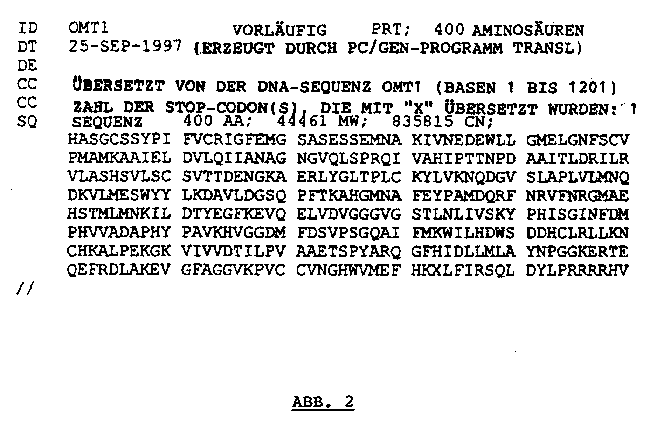

Die Erfindung umfaßt auch rekombinante Proteine mit der in Figur 2 dargestellten Aminosäuresequenz oder funktionellen Teilen hiervon. Unter "funktionellen Teilen" sind erfindungsgemäß solche Sequenzen des rekombinanten Proteins der Figur 2 zu verstehen, die die biologische PMT-Aktivität entfalten. Weiterhin umfaßt werden Proteine mit einer Aminosäuresequenz und Homologe oder Fragmente des Proteins, die die biologische PMT-Aktivität entfalten, deren Aminosäuresequenz sich von der DNA-Sequenz der Figur 1 oder deren Varianten in der oben beschriebenen Form ableiten.

Erfindungsgemäß beansprucht werden auch Prokaryontenzellen oder Eukaryontenzellen, die die erfindungsgemäße DNA in integrierter Form oder extrachromosomal auf einem Klonierungsvehikel enthalten. Beispiele für eine Prokaryontenzelle sind E.coli-Zellen, für eine Eukaryontenzelle Pflanzenzellen, in denen das PMT-Protein exprimierbar ist. Ein Beispiel für eine derartige Pflanzenzelle ist eine monokotyle oder eine dikotyle Pflanzenzelle.

Die Erfindung erstreckt sich auch auf transgene Pflanzen, die von einer derartigen transgenen Pflanzenzelle, die die erfindungsgemäße DNA-Sequenz enthält, abgeleitet sind. Die Erfindung erstreckt sich auch auf Teile dieser Pflanzen und deren Vermehrungsmaterial, beispielsweise Protoplasten, Pflanzenzellen, Kalli, Samen, Knollen oder Stecklinge sowie die Nachkommen dieser Pflanzen.

Erfindungsgemäß bereitgestellt wird ein Verfahren zur Herstellung transformierter Pflanzenzellen (einschließlich Protoplasten) und Pflanzen (einschließlich Pflanzenteile und Samen) zur Expression von rekombinantem Pinosylvin-3-O-methyltransferase-Protein oder einem funktionellen Teil hiervon, umfassend (a) das Einbringen einer DNA nach einem oder mehreren der vorhergehenden Ansprüche in eine Pflanzenzelle (einschließlich Protoplasten), und gegebenenfalls (b) Regenerieren aus den transformierten Pflanzenzellen (einschließlich Protoplasten) von vollständig transformierten Pflanzen, und gegebenenfalls (c) Gewinnen der gewünschten Pflanzenteile (einschließlich Samen) der so erhaltenen transformierten Pflanzen.

Bevorzugt wird das rekombinante PMT-Protein in einer solchen Menge exprimiert, daß durch die Methylierung von Pinosylvin ein zumindest teilweiser Schutz gegenüber durch pathogene Organismen verursachte Erkrankungen der Pflanzen bewirkt wird. Es kann ausreichend sein, daß lediglich die funktionellen Teile des rekombinanten Proteins exprimiert werden, um einen derartigen Schutz zu bewirken.

In einer weiteren bevorzugten Ausführungsform wird das rekombinante Protein oder ein funktioneller Teil hiervon zu Methylierung von Pinosylvin oder einem Derivat von Pinosylvin benutzt, wobei sich dieser Anwendungszweck auch auf pharmazeutische Einsatzgebiete oder sonstige Gebiete erstrecken kann, bei denen eine Methylierung von Pinosylvin von hoher Spezifität erforderlich ist.

Verfahren, um Fremd-DNA in Pflanzenzellen einzubringen und in diesen Fremdproteine zu exprimieren, somit transgene Pflanzenzellen um Pflanzen zu erzeugen, sind aus dem Stand der Technik bekannt.

Zur Vorbereitung der Einführung fremder Gene in höhere Pflanzen bzw. deren Zellen stehen eine große Anzahl von Klonierungsvektoren zur Verfügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird für die Transformation von E.coli-Zellen verwendet. Transformierte E.coli-Zellen werden in einem geeigneten Medium gezüchtet und anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation können die Plasmid-DNA gespalten und gewonnene DNA-Fragmente mit anderen DNA-Sequenzen verknüpft werden. Jede Plasmid-DNA-Sequenz kann in den gleichen oder anderen Plasmiden kloniert werden.

Für die Einführung von DNA in eine pflanzliche Wirtszelle steht eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichketien. Dabei können sowohl stabile als auch transiente Transformanten erzeugt werden.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig. Dem Fachmann sind die gängigen Selektionsmarker bekannt, und es stellt für ihn kein Problem dar, einen geeigneten Marker auszuwählen. Gebräuchliche Selektionsmareker sind solche, die den transformierten Pflanzenzellen Resistenz gegenüber einem Biozid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin u.a. vermitteln.

Je nach Einführungsmethode gewünschter Gene in die Pflanzenzelle können weitere DNA-Sequenzen erforderlich sein. Werden Z.B. für die Transformation der Pflanzenzelle das Ti- oder Ri-Plasmid verwendet, so muß mindestens die rechte Begrenzung häufig jedoch die rechte und linke Begrenzung der im Ti- und Ri-Plasmid enthaltenen T-DNA als Flankenbereich mit den einzuführenden Genen verbunden werden.

Werden für die Transformation Agrobakterien verwendet, muß die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Selektionsmarker-Gen und einen Linker oder Polylinker, welche von der rechten und linken T-DNA-Grenzregion eingerahmt werden. Sie können direkt in die Agrobakterien transformiert werden (Holsters et al., 1978, Molecular and General Genetics 163, 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.

Die Verwendung von T-DNA für die Transformation von Pflanzenzelle ist intensiv untersucht und ausreichend in EP 120 515; Hoekema in: The Binary Plant Vector System, Offsetdrokkerij Kanters B.V., Alblasserdam (1985) Chapter V; Fraley et al. (1993) Crit. Rev. Plant. Sci., 4, 1-46 und An et al. (1985) EMBO J. 4, 277-287 beschrieben worden.

Für den Transfer der DNA in die Pflanzenzelle können PflanzenExplantate zweckmäßigerweise mit Agrobacterium tumefaciens oder Agrobacterium rhizogenes kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die Regeneration der Pflanzen erfolgt nach üblichen Regenerationsmethoden unter Verwendung bekannter Nährmedien. Die so erhaltenten Pflanzen bzw. Pflanzenzellen können dann auf Anwesenheit der eingeführten DNA untersucht werden. Andere Möglichkeiten der Einführung fremder DNA unter Verwendung des biolistischen Verfahrens oder durch Protoplasten-Transformation sind bekannt (vgl. z.B. Willmitzer L. (1993) Transgenic Plants, in: Biotechnology, A Multi-Volume Comprehensive Treatise (H.J. Rehm, G. Reed, A. Pühler, P. Stadler, eds.) Vol. 2, 627-659, V.C.H. Weinheim - New York - Basel - Cambridge).

Während die Transformation dikotyler Pflanzen bzw. deren Zellen über Ti-Plasmid-Vektorsysteme mit Hilfe von Agrobacterium tumefaciens wohl etabliert ist, weisen neuere Arbeiten darauf hin, daß auch monokotyle Pflanzen bzw. deren Zellen der Transformation mittels Agrobacterium-basierender Vektoren sehr wohl zugänglich sind (Chan et al. (1993) Plant Mol. Biol. 22, 491-506; Hiei et al. (1994) Plant J. 6, 271-282; Deng et al. (1990) Science in China 33, 28-34; Wilmink et al. (1992) Plant Cell Reports 11, 76-80; May et al. (1995) Bio/Technology 13, 486-492; Conner und Domiss (1992) Int. J. Plant Sci. 153, 550-555; Ritchie et al. (1993) Transgenic Res. 2, 252-265).

Alternative Systeme zur Transformation von monokotylen Pflanzen bzw. deren Zellen sind die Transformationen mittels des biolistischen Ansatzes (Wan und Lemaux 81994) Plant Physiol. 104, 37-48; Vasil et al. (1993) Bio/Technology 11, 1553-1558; ritala et al. (1994) Plant Mol. Biol. 24, 317-325; Spencer et al. (19-90) Theor. Appl. Genet. 79, 625-631), die Protoplasten-Transformation, die Elektroporation von partiell permeabilisierten Zellen, die Einbringung von DNA mittels Glasfasern.

Die transformierten Zellen wachsen innerhalb der Pflanze in der üblichen Weise (siehe auch McCormick et al. (1986) Plant Cell Reports 5, 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften.

Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Ebenso können nach üblichen Methoden transgene Linien bestimmt werden, die für die neuen Nukleinsäuremoleküle homozygot sind und ihr phänotypisches Verhalten hinsichtlich einer vorhandenen bzw. nicht vorhandenen Ozon-Responsivität untersucht und mit dem von hemizygoten Linien verglichen werden.

Selbstverständlich können Pflanzenzellen, die die erfindungsgemäßen Nukleinsäuremoleküle enthalten, einschließlich Protoplasten, Kalli, Suspensionskulturen u.ä. weiterkultiviert werden.

Nachfolgend wird dargestellt, wie die erfindungsgemäße cDNA-Sequenz gewonnen wurde. Allerdings zeigte sich bei der Lösung der erfindungsgemäß gestellten Aufgabe, daß hier keineswegs ein linearer, einfacher Weg einzuschlagen war, sondern diverse unerwartete Schwierigkeiten aus dem Weg zu räumen waren, die für das Vorliegen einer erfinderischen Tätigkeit sprechen.

Zunächst ist festzuhalten, daß, wie bereits oben beschrieben wurde, lediglich die Existenz der Aktivität eines PMT-Enzyms bekannt war. Das Enzym selbst lag jedoch nicht in gereinigter, isolierter Form vor, sondern lediglich in einem Gemisch, verunreinigt mit anderen Proteinen. Bekannt waren lediglich drei Peptidsequenzen, die über eine zweidimensionale, eletrophoretische Auftrennung erhalten wurden, die deutlich die vielen weiteren verunreinigenden Proteine zeigte.

Durch Vergleich mit einem nativen Gel wurden in der Dissertation von Frau Dr. Trost zwei Spots ermittelt, denen eine PMT-Aktivität zuzuordnen war. Diese zwei Spots wurden partiell sequenziert und ergaben die in Tabelle 14 der Dissertation angegebenen Peptidsequenzen. Aus der Literatur ist jedoch bekannt, daß Methyltransferasen oft mehrere Substrate akzeptieren. Die beschriebene Enzymaktivität hätte somit durchaus auch einer anderen, bekannten Methyltransferase zugeordnet werden können, und es lag zum Zeitpunkt der Veröffentlichung der Dissertation kein zwangsläufiger Beweis für die Existenz einer spezifischen Pinosyylvin-3-0-methyltransferase vor.

Somit lag am Anmeldetag der vorliegenden Erfindung keine homogene PMT-Fraktion und damit kein homogenes PMT-Enzym vor. Bekannt waren lediglich PMT-Aktivitäten sowie die Aminosäuresequenzen von drei Peptiden, denen Enzyme mit PMT-Aktivität zuzuordnen waren.

Üblicherweise versucht man bei der Isolierung von Genen von Enzymen, die an sich bekannt sind, von solchen Peptidsequenzen auszugehen, die für das gesuchte Gen spezifisch sind. Auch im vorliegenden Fall wurde von den PMT-Peptidsequenzen der Tabelle 14 ausgegangen. Über RT-PCR aus Nadelgewebe wurde versucht, spezifische Sequenzen zu ermitteln. Dieser Isolierungsversuch führte jedoch nicht zum gewünschten Erfolg. Es wurde deshalb auf eine cDNA-Bank zurückgegriffen. Diese cDNA-Bank wurde mit degenerierten Primern gescreent, wobei Ausgangspunkt der Primer die PMT-spezifischen Peptidsequenzen waren. Diese häufig praktizierte und übliche Vorgehensweise, die der Fachmann anwenden würde, wenn ihm bereits die Existenz eines Enzyms bekannt ist, führte jedoch überraschenderweise nicht zum Erfolg. Obwohl der Fachmann von der möglichen Existenz einer PMT-Enzym-Aktivität wußte, war das übliche Isolierungsverfahren nicht zielführend.

Statt des oben beschriebenen Verfahrens, welches bereits zwei Fehlschläge umfaßte, wurde zur Lösung der erfindungsgemäß gestellten Aufgabe der Versuch unternommen, aus an sich bekannten Methyltransferase-Sequenzen hochkonservierte Regionen auszuwählen. Dieses Verfahren war um so weniger erfolgversprechend, als nicht von einer hohen Sequenzhomologie ausgegangen werden kannte. (Diese unterdurchschnittliche Sequenzhomologie wurde auch nachträglich dadurch bestätigt, daß zu ausgewählten OMT-Sequenzen eine maximale Identität von nur ca. 45 % ermittelt wurde (Vergleich Seite 3, 3. Absatz der Beschreibung der vorliegenden Erfindung)).

Ausgehend von einem Primer aus einer hockconservierten Region einer bekannten pflanzlichen O-methyltransferase wurden cDNA-Klone gescreent. Diese Klone enthielten natürlich insbesondere Sequenzen an sich bekannter pflanzlicher O-methyltransferasen, und es war nicht sicher, ob überhaupt die gesuchte PMT-Sequenz hierdurch gefunden werden konnte. Die ermittelten Klone wurden dann einem weiteren Screening-Verfahren unterzogen und mit den bekannten PMT-Oligopeptidsequenzen verglichen. Ein Klon konnte gefunden werden, der eine aus Tabelle 14 bekannte PMT-Oligopeptidsequenz enthielt. Klone, die einem möglichen PMT-Protein mit einem Molekulargewicht von 37 kDa hätten zugeordnet werden können, wurden nicht gefunden.

Die Problematik bei der erfindungsgemäß gewählten Vorgehensweise wird auch durch ersichtlich, daß selbst die zu Screening eingesetzten hoch-konservierten OMT-Bereiche beim erfindungsgemäß ermittelten PMT-Klon Abweichungen in den Aminosäuren zeigten, wobei sogar bei einem hoch-konservierten Bereich, der aus einem Pentapeptid bestand, die Abweichung in einer Aminosäure feststellbar war.

Da mit diesem Verfahren ausschließlich Teilsequenzen erhalten wurden, mußte in einem weiteren Verfahrensschritt mit Hilfe zusätzlicher PCR-Reaktionen die Isolierung des Vollängenklons durchgeführt werden. Auch hier war die Isolierung des Vollängenklons zunächst nicht erfolgreich. Das übliche PCR-Temperaturprogramm mußte wie folgt angepaßt werden, um ein positives Ergebnis zu erhalten: 1 min 94° C, 1,5 min 50° C, 2 min 72° C. Zusammengefaßt waren verschiedene Versuchsansätze notwendig, von denen nur einer zielführend war und auch nur zu einem Klon führte, der eine gesuchte PMT-Sequenz enthielt. Das Auffinden des erfindungsgemäß bereitgestellten PMT-Gens war somit alles andere als trivial und stellte den Fachmann vor diverse Schwierigkeiten, deren Lösungsmöglichkeiten für die vorliegende Aufgabenstellung speziell entwickelt und angepaßt werden mußten.

### cDNA-BANK ANALYSE:

Eine Ozon-induzierte cDNA Bank aus Kiefer (Wegener et al., Biochem Biophys Acta 1350: 247-252, 1997), kloniert in den Vektor pSport1 (Gibco) wurde zur Isolierung des PMT-Klons eingesetzt. Plasmid DNA von ca. 100.000 cDNA-Klonen wurde mittels des Midiprep-Kits (Qiagen) isoliert. Die Klonierung des PMT-Klons erfolgte mittels PCR. Als Primer wurde eine hoch-konservierte Region von bekannten pflanzlichen O-Methyltransferasen (5'-[C/T]TIGTIGA[C/T]GTIGGIGGIGG-3'), sowie der SP6-Primer (5'ATTTAGGTGACACTATAG-3'], enthalten in der pSport1-'Sequenz, ausgewählt. Amplifizierte Produkte wurden mittels Gelelektrophorese gereinigt, ausgeschnitten und eluiert.

### KLONIERUNG UND SEQUENZIERUNG:

Eluierte DNA-Fragmente wurden in den Vektor pGEM-T (Promega) ligiert und in kompetente E.coli Zellen (Promega) transferiert. Plasmid-DNA wurde wie oben beschrieben gereinigt und sequenziert. Die Sequenzierung erfolgte durch Thermo-Sequenase und dem radioaktiv markierten Terminator Cycle Sequencing Kit von Amersham. Die Sequenzierung erfolgte in beiden Richtungen an drei unabhängigen Klonen. Eine bekannte PMT-Peptidsequenz (ILD-TYEG) (Trost, Pinosylvin-3-O-methyltransferase aus Kiefer, Dissertation, LMU München, 1994) konnte gefunden werden.

### SUBKLONIERUNG UND EXPRESSION IN E.COLI:

Mittels PCR wurden an den 5'- und 3'-Enden des PMT1-Klons Ndel und Notl Schnittstellen eingefügt (5'-GGATTCCATATGGGATCGGCTTCC-3'; 5'-CATGTCGCGGCCGCCGTGGAAG-3'). PCR-Reaktionen wurden mit dem pGEM-T Plasmid, welches die PMT1 cDNA enthielt, durchgeführt. PCR-Produkte wurden gereinigt und in den Ndel/Notl geschnittenen Expressionsvektor pET21(a)+ (Novagen) eingefügt. Kompetente E.coli BL-21-Zellen (Novagen) wurden damit transformiert. Das Wachstum erfolgte bei 37°C, 220 UpM in 50 ml LB-Medium, enthaltend 100 µg/ml Ampicillin und 33 µg/ml Chloramphenicol. Bei einer optischen Kulturdichte von 0,6 (590 nm) wurde 1 mM IPTG zur Expressionsinduktion des Fusionsprotein zugegeben und anschließend für weitere 3 h kultiviert. Zellen wurden bei 5.000 x g geerntet und in 50 mM Tris-HCl pH 7,5; 2 mM DTT und 10% Glycerol resuspendiert. Zellyse erfolgte durch Ultraschall (4 x 30 Sek.) bei 0°C. Nach Zentrifugation bei 10.000 x g für 15 Min. bei 4°C erfolgte im Überstand die O-Methyltransferase-Aktivitätsbestimmung.

### ENZYMATISCHE TESTS:

50 µl Proteinextrakt wurden Jeweils mit Zimtsäure (CA), Quercitin (Q) und Pinosylvin (PS) in 0,1 mM Hepes-KOH pH 7,7 und 50µM ¹⁴CS-Adenoslymethionin für 30 Min. bei 30°C inkubiert. Für Cinnamoyl-CoA (CCoA) erfolgte die Inkubation in 20 µl 5N NaOH bei 56°C. Reaktionen wurden mit 20 µl 6 N HCl gestoppt. Die Produkte wurden mit Ethylacetat extrahiert, mittels eines Szintillatorzählers radioaktiv ausgewertet und über Dünnschichtchromatographie (TLC) analysiert. Für die TLC wurde auf Cellulose-Platten (Merck) aufgetrennt. Für CA, CCoA und PS wurde als Laufmittel 2% Ameisensäure und für Q 20% Essigsäure verwendet. Die getrockneten Platten wurden unter UV-Licht und Ammoniak-Dampf analysiert und autoradiographiert. Detektierte Spots wurden abgekratzt, in Methanol resuspendiert und zentrifigiert. Vom Überstand wurden die Jeweiligen UV-Spektren gemacht.

Die erfindungsgemäß ermittelten Sequenzen sind in den Abbildungen 1 und 2 dargestellt.
- Abb. 1:: zeigt die cDNA-Sequenz, und
- Abb. 2:: die hiervon abgeleitete Aminosäuresequenz der Pinosylvin-3-O-methyltransferase aus Pinus sylvestris.

In der Sequenz befindet sich ein Stopcodon an der Position 383 der Aminosäuresequenz, das mit "X" in der Abbildung 2 bezeichnet wurde.

Durch die Bereitstellung der für die Pinosylvin-3-O-methyltransferase kodierenden cDNA-Sequenz ist es möglich, Pinosylvin-3-O-methylether (PSM) in transgenen Pflanzen zu erzeugen. PSM hat neben Pinosylvin eine zentrale Stellung innerhalb der Phytoalexinen von Bäumen. PSM hat eine stark antimikrobielle Wirkung, die deutlich stärker ist als die von Pinosylvin (PS). Der Biosyntheseweg von PSM ist wie folgt:

Biosynthese von PSM:

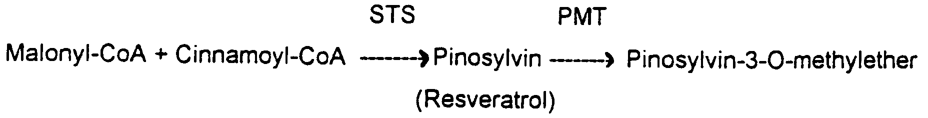

Nachfolgend wird ein Beispiel für die Expression des PMT-Proteins in transgenen Pflanzen beschrieben. Die Expression kann sowohl konstitutiv (35S-CaMV-Promotor) als auch unter der Kontrolle eines induzierbaren Promotors erfolgen. Gleichzeitig muß die Stilbensynthase (STS), welche die Vorläufersubstanz, Pinosylvin (PS), für die PMT liefert, exprimiert werden. Dies erfolgt ebenfalls durch Einbringung des STS-Gens in die gleiche transgene Pflanze. Die zu erwartende Akkumulation von PSM führt in den transgenen Pflanzen zu einem Schutz gegenüber Pathogenen und ist daher für die Landwirtschaft von Interesse.

Eine ähnliche Strategie wurde mit der Expression von Resveratrol in transgenen Pflanzen gezeigt: die STS aus Weinrebe wurde in Tabak und Reis exprimiert und führte dann zu einer erhöhten Resistenz gegenüber Pathogenen (Hain et al., Nature 361: 153-156 (1993); Stark-Lorenzen et al., Plant Cell Rep 16: 668-673, 1997). Da PSM wesentlich stärker antimikrobiell wirksam ist als PS, die analoge Verbindung zu Resveratrol, ist eine erhöhte Resistenz transgener Pflanzen gegenüber Pathogenen zu erwarten.

Die erfindungsgemäß beschriebene PMT-DNA-Sequenz eignet sich zur Expression von PMT-Enzym in pflanzlichen Substraten, in denen ihre Methylierungsaktivität einsetzbar ist. Derartige pflanzliche Substrate sind sowohl von landwirtschaftlichem (z.B. Expression in Kartoffel, Tomate oder Reis) als auch pharmazeutischem Interesse.

## Patentansprüche

1. Pinosylvin-3-O-methyltransferase-Gen und funktionelle Teile hiervon.

2. Pinosylvin-3-O-methyltransferase-Gen sowie funktionelle Teile hiervon nach Anspruch 1, erhältlich aus Kiefer (Pinus sylvestris).

3. Gereinigte und isolierte DNA mit der in Figur 1 dargestellten Pinosylvin-3-O-methyltransferase-Sequenz (PMT-Sequenz), die für ein pflanzliches PMT-Protein kodiert, oder ein funktioneller Teil der DNA.

4. DNA, die mit der in Figur 1 dargestellten PMT-Sequenz unter stringenten, bevorzugt hochstringenten Standard-Bedingungen hybridisierbar ist, die für ein pflanzliches PMT-Protein kodiert, oder ein funktioneller Teil der DNA.

5. DNA nach einem oder mehreren der vorhergehenden Ansprüche, welche aufgrund der Degeneration des genetischen Codes für dieselbe Aminosäuresequenz kodiert.

6. DNA nach einem oder mehreren der vorhergehenden Ansprüche, welche zumindest eine Deletion, Substitution, Addition und/oder Insertion eines oder mehrerer Nukleotide enthält, die für ein biologisch aktives PMT-Protein kodiert oder ein funktioneller Teil der DNA.

7. DNA nach einem oder mehreren der vorhergehenden Ansprüche, welche Introns enthält.

8. DNA nach einem oder mehreren der vorhergehenden Ansprüche, wobei der funktionelle Teil für ein biologisch aktives PMT-Qligopeptid kodiert oder eine für das PMT-Protein spezifische Regulationssequenz darstellt.

9. DNA nach einem oder mehreren der vorhergehenden Ansprüche aus Pinus sylvestris.

10. Rekombinantes DNA-Klonierungsvehikel, welches die in einem der vorhergehenden Ansprüche definierte DNA umfaßt.

11. DNA-Klonierungsvehikel nach Anspruch 10, der ein Expressionsvektor ist.

12. Rekombinante DNA mit einer DNA-Sequenz nach einem oder mehreren der vorhergehenden Ansprüche.

13. cDNA der Pinosylvin-3-O-methyltransferase.

14. Prokaryontenzelle oder Eukaryontenzelle, enthaltend eine DNA nach einem oder mehreren der vorhergehenden Ansprüche.

15. Prokaryontenzelle oder Eukaryontenzelle nach Anspruch 15, enthaltend die DNA nach einem oder mehreren der vorhergehenden Ansprüche in integrierter Form in der Wirts-DNA oder im DNA-Klonierungsvehikel.

16. Eukaryontenzelle nach Anspruch 14 oder 15, welche eine Pflanzenzelle ist.

17. Transgene Pflanze, die die DNA-Sequenz nach einem oder mehreren der vorhergehenden Ansprüche enthält sowie Teile dieser Pflanzen und deren Vermehrungsmaterial, wie Protoplasten, Pflanzenzellen, Kalli, Samen, Knollen oder Stecklinge, sowie die Nachkommen dieser Pflanzen.

18. Verwendung einer DNA-Sequenz nach einem oder mehreren der vorhergehenden Ansprüche zur Erzeugung transgener Pflanzen, die gegenüber pathogenen Organismen zumindest teilweise geschützt sind.

19. Rekombinantes Protein, das die in Figur 2 dargestellte Aminosäuresequenz oder funktionelle Teile hiervon umfaßt.

20. Rekombinantes Protein nach Anspruch 20, wobei eine oder mehrere der Aminosäuren deletiert oder substituiert sind und/oder eine oder mehrere Aminosäuren zusätzlich vorhanden sind, mit der Funktion eines biologisch aktiven PMT-Proteins.

21. Protein mit einer von der DNA-Sequenz nach einem oder mehreren der vorhergehenden Ansprüche abgeleiteten Aminosäuresequenz und Homologe oder Fragmente des Proteins, die die biologische PMT-Aktivität beibehalten.

22. Verwendung von rekombinantem Protein oder funktionellen Teilen hiervon nach einem oder mehreren der vorhergehenden Ansprüche zur Methylierung von Pinosylvin oder einem Derivat hiervon.

23. Verfahren zur Herstellung transformierter Pflanzenzellen (einschließlich Protoplasten) und Pflanzen (einschließlich Pflanzenteile und Samen) zur Expression von rekombinantem Pinosylvin-3-O-methyltransferase-Protein oder einem funktionellen Teil hiervon, umfassend
(a) das Einbringen einer DNA nach einem oder mehreren der vorhergehenden Ansprüche in eine Pflanzenzelle (einschließlich Protoplasten), und gegebenenfalls
(b) Regenerieren aus den transformierten Pflanzenzellen (einschließlich Protoplasten) von vollständig transformierten Pflanzen, und gegebenenfalls
(c) Gewinnen der gewünschten Pflanzenteile (einschließlich Samen) der so erhaltenen transformierten Pflanzen.

24. Verfahren nach Anspruch 23,
dadurch gekennzeichnet, daß
das rekombinante PMT-Protein in einer solchen Menge exprimiert wird, daß durch die Methylierung von Pinosylvin ein zumindest teilweiser Schutz gegenüber durch pathogene Organismen verursachte Erkrankungen der Pflanzen bewirkt wird.

25. RNA-Sequenz, die sich von einer DNA-Sequenz nach einem oder mehreren der vorhergehenden Ansprüche ableitet.


SEQUENZPROTOKOLL

<110> GSF-Forschungszentrum f. Umwelt und Gesundheit

<120> Protein- und DNA-Sequenz der
      Pinosylvin-3-O-methyltransferase (PMT)

<130> P11520

<140> DE 198 36 774.0
<141> 1998-08-13

<160> 7

<170> PatentIn Ver. 2.1

<210> 1
<211> 1201
<212> DNA
<213> Pinus sylvestris

<400> 1
cacgcgtccg ggtgctcctc ttacccaatt tttgtgtgta gaattggatt cgaaatggga 60
tcggcttccg aaagctcaga gatgaatgcg aagattgtga atgaagatga atggcttctg 120
ggcatggaac tcgggaattt ctcctgcgtt cctatggcta tgaaggcagc cattgagctc 180
gatgtcctcc aaatcatagc aaatgccggc aatggcgtcc agctctcgcc cagacaaatc 240
gttgcccaca ttccgaccac gaaccccgac gctgccatta cacttgatcg gatcctcaga 300
gtgttagcca gccattcggt tctgagctgc tctgtgacca cggacgagaa tggcaaggcc 360
gagaggctct atggcctcac tcctctctgc aaatatctgg tcaagaacca ggatggagtt 420
tctctggctc cattggtgtt gatgaatcaa gataaggtgt tgatggagtc ctggtattat 480
ctcaaggacg cggtgctgga tggttcccag ccattcacca aagcccatgg aatgaatgcg 540
ttcgagtacc cagccatgga ccagagattc aaccgggttt tcaaccgggg aatggctgag 600
cattctacca tgttgatgaa caagattctg gatacttacg agggttttaa ggaggttcag 660
gaattggtgg atgtgggagg aggagttggg tcgaccctca atctcatagt ctcgaagtat 720
ccccacattt caggaatcaa ctttgacatg ccccatgtgg tggccgatgc tcctcactat 780
ccagctgtga aacatgtggg tggagacatg tttgatagcg taccaagtgg ccaagctatc 840
tttatgaagt ggatcctgca tgattggagc gatgatcatt gcttgaggct tttgaagaat 900
tgtcacaagg cattgccaga gaaagggaaa gtgattgtgg tggacaccat tctccctgtt 960
gctgcagaga catctcccta tgctcgccag ggatttcata tagatctgtt gatgttggcg 1020
tacaatccag gaggcaagga gcgcacagag caagaatttc gagatttggc taaagaggtg 1080
ggatttgcag gtggcgttaa acctgtctgt tgtgtcaatg gacattgggt aatggaattc 1140
cacaagtaac tcttcattag atcgcagttg gattatcttc cacgtcggcg gcgacatgtt 1200
a                                                                 1201

<210> 2
<211> 20
<212> DNA

<213> Künstliche Sequenz

<220>
<223> Beschreibung der künstlichen Sequenz:
      Oligonukleotid

<400> 2
ytngtngayg tnggnggngg                                                    20


<210> 3
<211> 18
<212> DNA
<213> Künstliche Sequenz

<220>
<223> Beschreibung der künstlichen Sequenz:
      Oligonukleotid

<400> 3
atttaggtga cactatag                                                      18


<210> 4
<211> 24
<212> DNA
<213> Künstliche Sequenz

<220>
<223> Beschreibung der künstlichen Sequenz:
      Oligonukleotid

<400> 4
ggattccata tgggatcggc ttcc                                               24


<210> 5
<211> 22
<212> DNA
<213> Künstliche Sequenz

<220>
<223> Beschreibung der künstlichen Sequenz:
      Oligonukleotid

<400> 5
catgtcgcgg ccgccgtgga ag                                                 22

<210> 6
<211> 7
<212> PRT
<213> Pinus sylvestris

<400> 6
Ile Leu Asp Thr Tyr Glu Gly
1               5

<210> 7
<211> 400
<212> PRT
<213> Pinus sylvestris

<400> 7
His Ala Ser Gly Cys Ser Ser Tyr Pro Ile Phe Val Cys Arg Ile Gly
1               5                   10                  15

Phe Glu Met Gly Ser Ala Ser Glu Ser Ser Glu Met Asn Ala Lys Ile
            20                  25                  30

Val Asn Glu Asp Glu Trp Leu Leu Gly Met Glu Leu Gly Asn Phe Ser
        35                  40                  45

Cys Val Pro Met Ala Met Lys Ala Ala Ile Glu Leu Asp Val Leu Gln
    50                  55                  60

Ile Ile Ala Asn Ala Gly Asn Gly Val Gln Leu Ser Pro Arg Gln Ile
65                  70                  75                  80

Val Ala His Ile Pro Thr Thr Asn Pro Asp Ala Ala Ile Thr Leu Asp
                85                  90                  95

Arg Ile Leu Arg Val Leu Ala Ser His Ser Val Leu Ser Cys Ser Val
            100                 105                 110

Thr Thr Asp Glu Asn Gly Lys Ala Glu Arg Leu Tyr Gly Leu Thr Pro
        115                 120                 125

Leu Cys Lys Tyr Leu Val Lys Asn Gln Asp Gly Val Ser Leu Ala Pro
    130                 135                 140

Leu Val Leu Met Asn Gln Asp Lys Val Leu Met Glu Ser Trp Tyr Tyr
145                 150                 155                 160

Leu Lys Asp Ala Val Leu Asp Gly Ser Gln Pro Phe Thr Lys Ala His
                165                 170                 175

Gly Met Asn Ala Phe Glu Tyr Pro Ala Met Asp Gln Arg Phe Asn Arg
            180                 185                 190

Val Phe Asn Arg Gly Met Ala Glu His Ser Thr Met Leu Met Asn Lys
        195                 200                 205

Ile Leu Asp Thr Tyr Glu Gly Phe Lys Glu Val Gln Glu Leu Val Asp
    210                 215                 220

Val Gly Gly Gly Val Gly Ser Thr Leu Asn Leu Ile Val Ser Lys Tyr
225                 230                 235                 240

Pro His Ile Ser Gly Ile Asn Phe Asp Met Pro His Val Val Ala Asp
                245                 250                 255

Ala Pro His Tyr Pro Ala Val Lys His Val Gly Gly Asp Met Phe Asp
            260                 265                 270

Ser Val Pro Ser Gly Gln Ala Ile Phe Met Lys Trp Ile Leu His Asp
        275                 280                 285

Trp Ser Asp Asp His Cys Leu Arg Leu Leu Lys Asn Cys His Lys Ala
    290                 295                 300

Leu Pro Glu Lys Gly Lys Val Ile Val Val Asp Thr Ile Leu Pro Val
305                 310                 315                 320

Ala Ala Glu Thr Ser Pro Tyr Ala Arg Gln Gly Phe His Ile Asp Leu
                325                 330                 335

Leu Met Leu Ala Tyr Asn Pro Gly Gly Lys Glu Arg Thr Glu Gln Glu
            340                 345                 350

Phe Arg Asp Leu Ala Lys Glu Val Gly Phe Ala Gly Gly Val Lys Pro
        355                 360                 365

Val Cys Cys Val Asn Gly His Trp Val Met Glu Phe His Lys Xaa Leu
    370                 375                 380

Phe Ile Arg Ser Gln Leu Asp Tyr Leu Pro Arg Arg Arg Arg His Val
385                 390                 395                 400